# EUROPEAN PATENT APPLICATION

(11) **EP 4 624 485 A1**
(43) Date of publication of application: **01.10.2025**
(21) Application number: 24382324.2
(22) Date of filing: 25.03.2024
(51) Int. Cl.: C07K 7/08, A61K 47/64, G01N 33/68, A61P 35/00

(54) **PEPTIDE THAT TARGETS MACROPHAGES AND USES THEREOF**

(71) Applicant: Consejo Superior De Investigaciones Científicas, 28006 Madrid (ES); University of Tartu, 50090 Tartu (EE); Universidad Nacional de General San Martín, B1650 San Martín, Provincia de Buenos Aires (AR)
(72) Inventor: Scodeller, Pablo David, Madrid (ES); Teesalu, Tambet, Tartu (EE); Asciutto, Eliana Karina, Buenos Aires (AR); Lepland, Anni, Tartu (EE)
(74) Representative: Pons IP

(57) **Abstract**

The invention relates to a peptide that targets macrophages, conjugates comprising said peptide, and therapeutic and diagnostic uses thereof. More particularly, the present invention refers to a peptide that targets CD206 which is expressed in M2-macrophages.

## Description

The invention relates to a peptide that targets macrophages, conjugates comprising said peptide, and therapeutic and diagnostic uses thereof. Therefore, the present invention is within the field of biomedicine.

### BACKGROUND ART

Established solid tumors exploit the immunosuppressive and angiogenic nature of anti-inflammatory macrophages (M2 type) to progress and metastasize. Additionally, by secreting cytokines that skew macrophages to M2, tumors can expand this CD206+ TAM compartment, reaching up to 60% of the hematopoietic pool in the tumor. Because of their major contribution in progression, depletion of CD206+ TAMs (Binnewies, M., et al., Cell Reports 2021, 37 (3), 109844) or modulation (Viitala, M., et al., Clinical Cancer Research 2019, 25 (11), 3289-3303) towards an inflammatory (M1) phenotype has become a popular approach to potentiate immune checkpoint blockade in breast cancer.

The macrophage mannose receptor 1 (CD206, encoded by the gene MRC1), is over-expressed on anti-inflammatory macrophages and protumoral and pro-metastatic subpopulation of TAMs. Targeting CD206 has applications in cancer therapy (Conniot, J. et al., Nat Nanotechnol 2019, 14 (9), 891-901) and diagnosis of early cancer, as an increase in the number of CD206+ cells in the lymph nodes correlates with onset of relapse in head and neck cancer in humans (Azad, A. K., et al., The Journal of Immunology 2015). Most chemically synthesized systems designed to target CD206 utilize mannose as the recognition moiety. However, as the affinity of mannose is on the low millimolar range, this dictates the necessity for multivalent presentation in order to achieve binding.

Short linear targeting peptides are selective ligands, that can guide therapeutic or imaging cargos to tumors (Scodeller, P., et al.,Scientific Reports 2017, 7 (1); Lepland, A., et al., Mol. Pharmaceutics 2020, 17 (7), 2518-2531). Their relatively low affinity renders them attractive as targeting elements to coat on nanoparticles, as binding is increased through avidity, without observing binding site barrier effects.

For monovalent 1:1 peptide-drug conjugates however, low affinity of the targeting peptide is a limitation. Short unconstrained peptides have high conformational freedom which translates into poor affinity and proteolytic degradation. Moreover, the administration of therapeutic peptides is limited to some routes such as intravenous administration and it would be interesting to expand it to other routes such as oral administration, which represents an easy and less invasive route for administering therapeutics. There is a need in the prior art to provide modified short peptides that show a higher affinity, stability and can be administered by other routes for a therapeutic or diagnostic purpose.

### DESCRIPTION OF THE INVENTION

The present inventors have engineered the CD206-binding peptide mUNO (SEQ ID NO: 3: CSPGAK), with the aim of enhancing affinity and proteolytic stability. To this end, the present inventors exploited the bounties of the Sunflower Trypsin Inhibitor I peptide (SFTI-1, SEQ ID NO: 4: GRCTKSIPPICFPD), a plant-derived peptide, composed of two loops separated by a disulfide bond; the largest loop (referred to as the primary loop) of which, inhibits the activity of serine proteases, including trypsin and pepsin. Also, SFTI-1 is conformationally constrained by a disulfide bond, three proline residues and several hydrogen bonds. The other loop, referred to as the cyclization loop has been modified by present inventors to introduce foreign peptides without affecting the enzyme-inhibiting activity or the oral availability.

The present inventors developed new engineered CD206-binding peptide, with a 15-fold higher affinity and 5-fold higher stability (examples 2 and 3; Fig. 2 and Fig. 3) that can advantageously be delivered not only intravenously, but also orally to a subject, and it is also able to selectively target tissues that comprise CD206+ macrophages (example 4; Fig. 4). The present inventors also reported a Verteporfin conjugate of this engineered CD206-binding peptide that can be used for light-dependent depletion of CD206+ macrophages or light-independent modulation of CD206+ macrophages to a mixed M1/M2 TAM (tumor-associated macrophages) phenotype with associated therapeutic benefit (example 5; example 7; example 8; Fig. 5; Fig. 7; Fig. 8). Moreover, the inventors also found that the engineered CD206-binding peptide shows anti-leishmanial activity (example 9; Fig. 9).

Thus, in one aspect, the present invention relates to a peptide, hereinafter "the peptide of the invention", that comprises, or consists of, the amino acid sequence SEQ ID NO: 1: CTKSIPPICSPGAK.

As used herein, the terms "peptide", "polypeptide" and "protein" are used interchangeably and refer to a polymer of amino acid residues linked via peptide bonds, and which may be composed of two or more polypeptide chains. The terms "polypeptide", "protein" and "peptide" refer to a polymer of at least two amino acid monomers joined together through amide bonds.

In a preferred embodiment, alone or in combination with the other preferred embodiments, the peptide of the invention has a length of no more than 50 amino acids, more preferably, no more than 40 amino acids, no more than 30 amino acids, and even more preferably no more than 20 amino acids.

The peptide of the invention results from the fusion of, by means of a peptide bond, the amino acids conforming the primary loop of the SFTI-1 peptide and the mUNO peptide.

The cysteine residues of the peptide of the invention (specifically, cysteine residues at positions 1 and 9 of SEQ ID NO: 1) comprise -SH groups that may chemically react leading to the formation of a disulfide bond, that results in the formation of a loop that is equivalent to the primary loop of the SFTI-1 peptide.

In the present invention, the term "disulfide bond" refers to a chemical bond, preferably a covalent bond, formed between two sulfhydryl groups; more preferably, the term disulfide bond refers to a covalent bond between the sulfhydryl groups of two cysteines (comprised in a peptide, preferably the peptide of the invention). The formation of disulfide bonds in peptides is known of the skilled person, by means of methods that comprise the oxidation of sulfhydryl groups of cysteines using an oxidizing agent such as, but not limited to, dimethyl sulfoxide (DMSO).

In a preferred embodiment, alone or in combination with the other preferred embodiments, the peptide of the invention is cyclic.

As used herein in reference to a peptide, the term "cyclic" refers to a structure including an intramolecular bond between two non-adjacent amino acids forming a loop or cycle in the structure of said peptide. The cyclization can be effected through a covalent or non-covalent bond. Intramolecular bonds include, but are not limited to, backbone to backbone, side-chain to backbone and side-chain to side-chain bonds. Cyclic peptides may be monocyclic or polycyclic, depending on whether there is one or two or more intramolecular bonds, respectively.

In another preferred embodiment of the peptide of the invention, alone or in combination with the other preferred embodiments, the cysteine at position 1 (SEQ ID NO: 1) and the cysteine at position 9 (SEQ ID NO: 1) are linked through, or form, a disulfide bond.

In another preferred embodiment, alone or in combination with the other preferred embodiments, the peptide of the invention is cyclic, preferably monocyclic, wherein the cysteine at position 1 (SEQ ID NO: 1) and the cysteine at position 9 (SEQ ID NO: 1) are linked through, or form, a disulfide bond. As a result, a cyclization loop is formed.

The present inventors also added an amino acid to the N-terminal end of the peptide of SEQ ID NO: 1. Specifically, the present inventors added a glycine amino acid residue to have a flexible spacer between the cyclization loop and a therapeutic agent or a detectable agent that may be linked to the peptide.

Thus, in another preferred embodiment, alone or in combination with the other preferred embodiments, the peptide of the invention comprises, preferably consists of, the amino acid sequence SEQ ID NO: 2: GCTKSIPPICSPGAK.

In another preferred embodiment, alone or in combination with the other preferred embodiments, the peptide of the invention is cyclic, preferably monocyclic, wherein the cysteine at position 2 of SEQ ID NO: 2 and the cysteine at position 10 of SEQ ID NO: 2 are linked through, or form, a disulfide bond. As a result, a cyclization loop is formed.

The glycine residue at the N-terminal end makes the peptide of the invention to be more constrained and contributes to the binding with CD206.

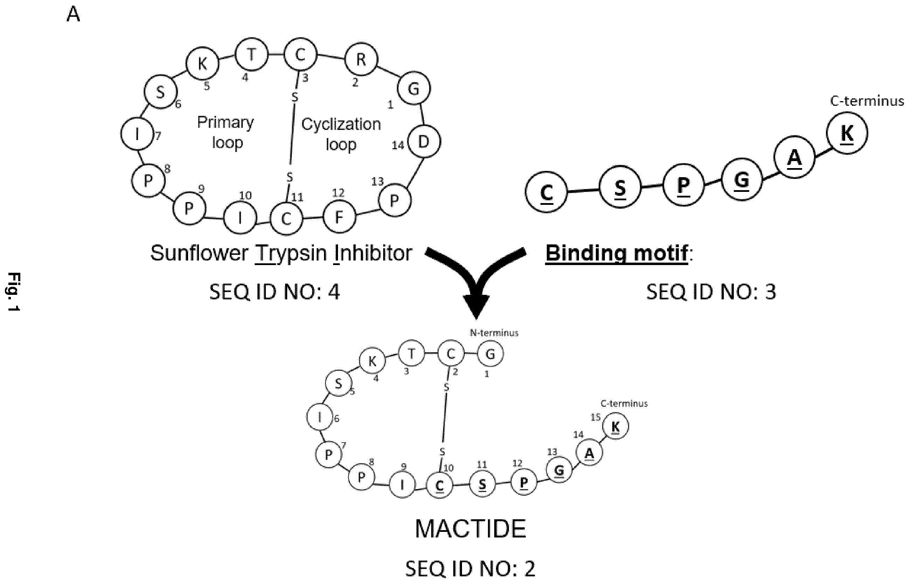

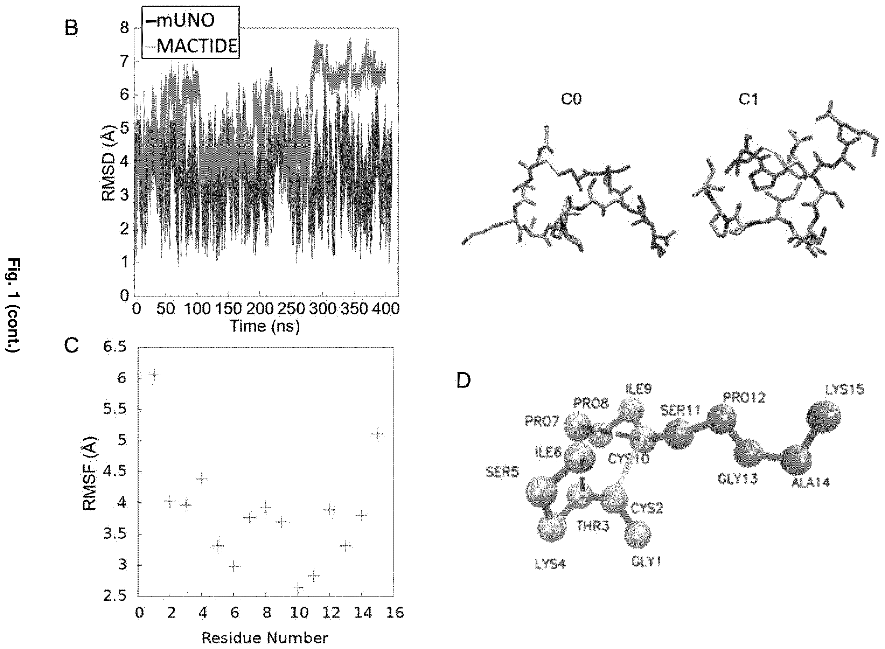

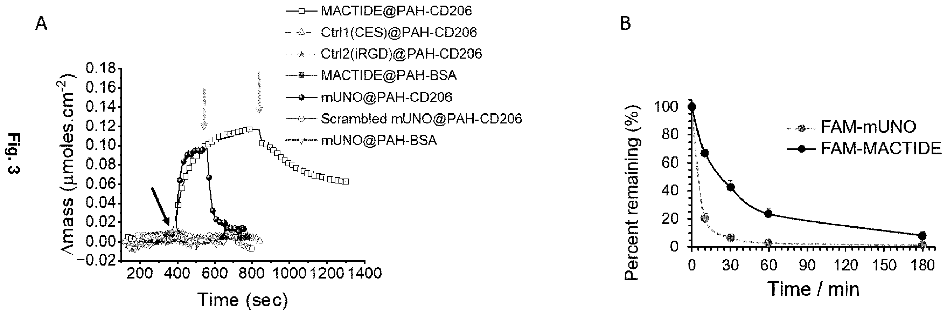

The above-mentioned cyclization loop (Fig. 1) provides the peptide of the invention with more proteolytic stability (Fig. 3B).

In the present invention the term "proteolytic stability" when referring to a peptide (i.e. the peptide of the invention) refers to the capacity of said peptide to maintain chemical structure in presence of compounds or conditions that act hydrolysing the peptide bonds, wherein said compounds may comprise proteolytic enzymes such as protases present in biological tissues. The proteolytic stability of a peptide may be represented as the half-life of said peptide, preferably represented in minutes or hours. The peptide of the invention showed a higher proteolytic stability (higher half-life value) compared to the peptide of SEQ ID NO: 3 alone (mUNO without the amino acid portion of the SFTI-I). The proteolytic stability of the peptide of the invention can be determined as in example 3.

Surprisingly the peptide of the invention showed higher affinity to bind the CD206 (Fig. 3A) compared to the peptide of SEQ ID NO: 3 alone (mUNO without the amino acid portion of the SFTI-I).

In the present invention, the term "affinity" or "binding affinity" refers to the strength of the total number of non-covalent interactions between a single binding site in a molecule X (e.g., a protein receptor, preferably CD206) and its binding partner Y (e.g., peptide that binds a protein receptor, preferably the peptide of the invention). Unless otherwise indicated, in the context of the present invention, the term "binding affinity" refers to an affinity that reflects a 1:1 interaction between members of a binding pair (e.g., protein-receptor). The affinity of a molecule X for a partner Y can usually be represented by the dissociation constant (Kd), which means the ratio of the dissociation rate constants and the association rate constant.

Thus, the equivalent affinities can represent different rate constants if the ratio of the rate constants remains unchanged.

The peptide of the invention specifically binds the CD206 receptor. In the present invention, the term "specifically binds" refers to the non-covalent physical association between two molecules or molecular entities, i.e. the peptide of the invention and the CD206. It is considered that the peptide of invention specifically binds to CD206, wherein the binding strength between the peptide of the invention and CD206 is at least 10 times, at least 15 times, at least 20 times higher, at least 50 times, at least 75 or at least 100 times higher than the binding strength of CD206 and any irrelevant molecule. It is also considered that the peptide of the invention specifically binds CD206 wherein the dissociation constant has a value of 10⁻³ or less, 10⁻⁴ or less, 10⁻⁵ or less, 10⁻⁶ or less, 10⁻⁷ or less, 10⁻⁸ or less, 10⁻⁹ or less, 10⁻¹⁰ or less, 10⁻¹¹ or less, or 10⁻¹¹ or less, under the employed conditions, such as physiological conditions, culture conditions or conditions that allow cell surviving.

Affinity and the specific union or bond can be measured by conventional methods known in the art, including, but not limited to, Surface plasmon resonance (SPR) or Quartz Crystal Microbalance (QCM), preferably QCM; preferably as measured in the examples of the present invention.

The peptide of the invention targets to cells, tissues or organs, containing cells that express CD206 on their surface.

The protein "CD206", "Macrophage Mannose Receptor 1" or "Macrophage Mannose Receptor 1-Like Protein 1", used interchangeably, refers to a protein receptor (uniprot reference for human CD206: P22897-1, SEQ ID NO: 5) that is encoded by the gene MRC1 (ensemble reference for gene MRC1: ENSG00000260314). The protein receptor CD206 is a membrane receptor that is a C-type lectin primarily present on the surface of macrophages, immature dendritic cells and liver sinusoidal endothelial cells, but is also expressed on the surface of skin cells such as human dermal fibroblasts and keratinocytes, which plays a role in both innate and adaptive response of the immune system.

Amino acid sequence of CD206, SEQ ID NO: 5:

Macrophages may present two phenotypes: M1 and M2. M1 macrophages are a pro-inflammatory, whereas M2 macrophages are anti-inflammatory. CD206 is overexpressed in M2 macrophages. The balance of M1/M2 macrophages is quite relevant in some diseases such as cancer, wherein the macrophages comprised in the tumor microenvironment (tumor associated macrophages, TAMs) play an important role in the progression of the diseases. M1 macrophages, which are pro-inflammatory act as tumor-inhibiting macrophages, and M2 macrophages, which are anti-inflammatory, act as tumor-supporting (promote the progression and metastasis).

The present inventors also found that the peptide of the invention can promote the production of cytokines corresponding to the M1 phenotype. Moreover, the peptide of the invention, which binds the CD206 present on the surface of M2 macrophages, is useful to target active agents, such as detectable agents or therapeutic agents, showing interesting features for the treatment and/or diagnosis of diseases related to M2 phenotype of macrophages.

Thus, in another aspect, the present invention relates to a conjugate, hereinafter "the conjugate of the invention", that comprises the peptide of the invention linked to a detectable agent or a therapeutic agent.

In the present invention, the term "conjugate" refers to a compound formed by the link of at least two or more chemical compounds (i.e. the peptide of the invention and a detectable agent or a therapeutic agent). In a conjugate the at least two chemical compounds may be linked by any type of chemical bond, preferably a covalent or a non-covalent bond.

The term "covalent bond" refers to a chemical bond that involves the sharing of electrons to form electron pairs between atoms, an example of covalent bond is the peptide bond. The term "non-covalent bond" refers to a chemical bond that, unlike covalent bonds, does not involve sharing of electrons between atoms. Non-covalent interactions can be ionic interactions or electrostatic interactions, Van der Waals interactions, hydrophobic interactions, hydrogen bonds.

In a preferred embodiment of the conjugate of the invention, alone or in combination with the other preferred embodiments, the peptide of the invention is linked by a covalent bond to a detectable agent or a therapeutic agent.

In another preferred embodiment of the conjugate of the invention, alone or in combination with the other preferred embodiments, the peptide of the invention is directly linked or indirectly linked, preferably by covalent bonds, to a detectable agent or a therapeutic agent.

The term "directly linked" means that the therapeutic or detectable agent is linked to any of the atoms or functional groups of the peptide of the invention, preferably to the N-terminal end, C-terminal end, or any other amino (-NH₂), carboxy (-COOH), sulfhydryl (-SH) or reactive groups of the peptide of the invention, preferably by covalent bonds.

The expressions "indirectly linked" or "linked through a linker", used interchangeably, mean that peptide of the invention and therapeutic or detectable agent are bound through a linker molecule functioning as bridge between the peptide of the invention and the targeting element. Therefore, when the peptide of the invention and the therapeutic or detectable agent are linked through a linker, they are not contiguously linked, meaning that the therapeutic or detectable agent is linked to the linker and the linker is linked to the peptide of the invention, preferably to the N-terminal end, C-terminal end, or any other amino (-NH₂), carboxy (-COOH), sulfhydryl (-SH) or reactive groups of the peptide of the invention, preferably by covalent bonds.

Thus, in a preferred embodiment of the conjugate of the invention, alone or in combination with the other preferred embodiments, the peptide of the invention is linked to a detectable agent or a therapeutic agent thought a linker, preferably by covalent bonds.

As used herein, the term "linker" refers to a chemical entity composed of at least one atom (such as carbon, oxygen, nitrogen, sulphur, phosphorous and combinations thereof), that links at least two other entities together. In the case of the invention, the two entities linked through a linker are the peptide of the invention and a targeting element. The linker may be selected from peptidyl linkers, non-peptidyl linkers or a combination thereof.

Thus, in a preferred embodiment of the conjugate of the invention, alone or in combination with the other preferred embodiments, the peptide of the invention is linked to the linked to a detectable agent or a therapeutic agent thought a linker, preferably by covalent bonds, wherein the linker is selected from a peptidyl linker non-peptidyl linkers or a combination thereof.

In the present invention, the "peptidyl linker" refers to an amino acid or peptide that consists of 2 to 50 amino acids in length, preferably 2 to 30 amino acids, more preferably 2 to 20 amino acids, more preferably 2 to 10 amino acids; preferably the peptidyl linker comprises amino acids selected from: glycine, serine, threonine and combinations thereof.

The term "non-peptidyl linker" refers to a linker based on straight or branched chain hydrocarbons or polyethylene glycols of varying lengths. These may incorporate other groups to effect solubility, rigidity, isoelectric point, such as aromatic or nonaromatic rings, halogens, ketones, aldehydes, esters, sulfonyls, phosphate groups, and so on. Examples of non-peptidyl linkers include, without limitation, 6-aminohexanoic acid (Ahx), sulfosuccinimidyl 4-(N-maleimidomethyl)cyclohexane-1-carboxylate, 4-(N-maleimidomethyl)cyclohexane-1-carboxylic 3-sulfo-n-hydroxysuccinimide ester or succinimidyl-4-(N-maleimidomethyl)cyclohexane-1-carboxy-(3-Am idoCaproate).

The peptide of the invention is able to target a therapeutic agent to cells, tissues or organs that contain cells that express CD206 on their surface, which allows a more effective targeted therapy for diseases or conditions wherein cells expressing CD206 are implicated, i.e. macrophages expressing CD206.

In the present invention, the term "therapeutic agent" refers to any compound or mixture of compounds which produces a physiological result or modulates a biological process, e.g., a beneficial effect for the treatment or prevention of a disease or condition. Examples of therapeutic agents that may be part of the conjugate of the invention include, without limitation, small molecules (molecules having a molecular weight ≤1000 daltons), deoxyribonucleic acids (DNA), ribonucleic acids (RNA), peptides, proteins, chemotherapeutic agents, radiopharmaceutical compounds or antibodies.

Thus, in another preferred embodiment of the conjugate of the invention, alone or in combination with the other preferred embodiments, the therapeutic agent is selected from the list consisting of: a photosensitizer compound, a small molecule, a DNA, a RNA, a peptide, a protein, a chemotherapeutic agent, a radiopharmaceutical compound, an antibody and any combination thereof; preferably wherein the RNA is selected from a non-coding RNA (ncRNA) or a messenger RNA (mRNA); more preferably wherein the ncRNA is selected from the list consisting of, but not limited to, micro-RNA (miRNA), small interfering-RNA (siRNA), long non coding RNA (IncRNA), antisense RNA or guide RNA (gRNA).

In the present invention the term "small molecule", refers to a any therapeutic small molecule having a molecular weight ≤ 1000 daltons.

In the present invention, the term "photosensitizer" or "photosensitizer compound", used interchangeably, refers to a compound that when irradiated by light produces a photochemical or phytotoxic effect on a cell, i.e., produce reactive species such as reactive oxygen species. Photosensitizers may show inherent toxic effects without the need of irradiation with light. The production of reactive species may be used for photodynamic therapy. Examples of photosensitizers include, but are not limited to, verteporfin, 5-aminolevulinic acid (5-ALA), chlorin e6, IRDye700DX or Porfimer sodium (Photofrin).

Photodynamic therapy (PDT) is a two-stage therapy that first comprises administering a photosensitiser to a subject and second, the photosensitizer is activated by light irradiation at a specific wavelength, preferably, laser irradiation at a wavelength ranging from 415 to near infrared (near infrared ranges from 780nm to 2500 nm), preferably laser irradiation at a wavelength ranging from 415 nm to 690 nm. The light irradiation may be directed to a specific part of the body of the subject, such as a part that is known to comprise a tumor tissue.

In another preferred embodiment of the conjugate of the invention, alone or in combination with the other preferred embodiments, the therapeutic agent is a photosensitizer selected form the list consisting of verteporfin, 5-aminolevulinic acid (5-ALA), chlorin e6, IRDye700DX and Porfimer sodium (Photofrin).

In another preferred embodiment of the conjugate of the invention, alone or in combination with the other preferred embodiments, the therapeutic agent is a photosensitizer wherein said photosensitizer is verteporfin. Verteporfin has the structure represented as the formula (I):

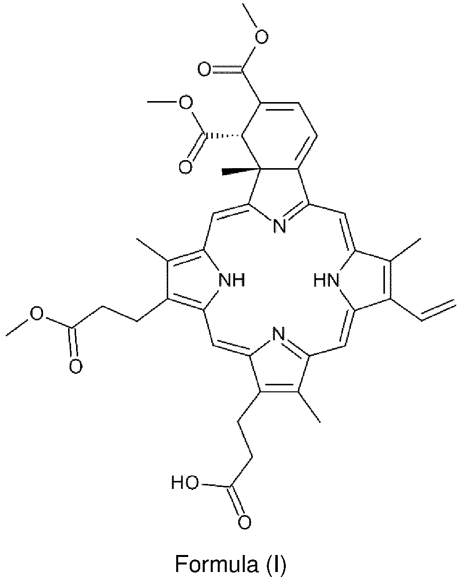

In another preferred embodiment of the conjugate of the invention, alone or in combination with the other preferred embodiments, the therapeutic agent is verteporfin, wherein the conjugate has the formula (II):

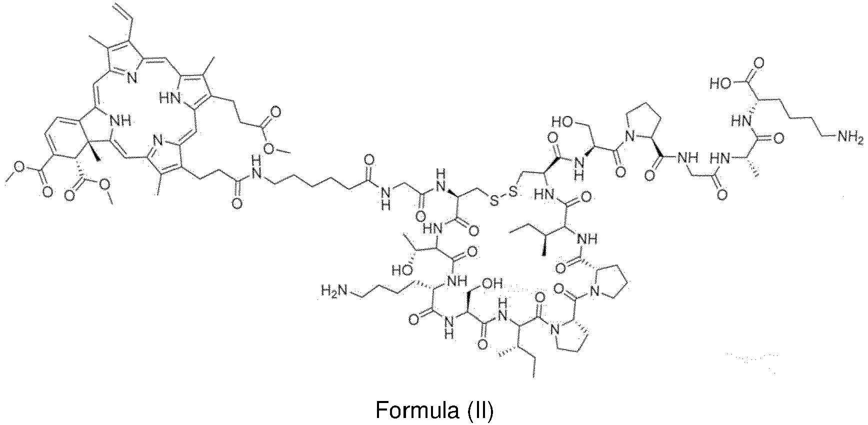

The peptide of the invention is able to target a detectable agent to cells, tissues or organs that contain cells that express CD206 on their surface, which allows to mark these cells, tissues or organs for detection or diagnostic purposes.

In the present invention, the term "detectable agent" or "detectable label", used interchangeably, refers to a compound that can emit a detectable signal; examples of detectable agents include, without limitation, fluorophores, chromophore, radionuclide, enzymes, metals, barium sulfate, ferromagnetic compounds, paramagnetic compounds, diamagnetic compounds or combinations thereof.

Thus, in another preferred embodiment of the conjugate of the invention, alone or in combination with the other preferred embodiments, the detectable agent is selected form the list consisting of fluorophore, a radionuclide, an enzyme (such as Hyaluronidase), a metal, barium sulphate, a ferromagnetic compound, a paramagnetic compound, a diamagnetic compound and any combination thereof.

Examples of fluorophores include, but are not limited to, near Infrared probes such as 800CW, Alexa-Fluor dyes (e.g., Alexa Fluor@ 350, Alexa Fluor^{®} 405, Alexa Fluor@ 430, Alexa Fluor@ 488, Alexa Fluor@ 500, Alexa Fluor@ 514, Alexa Fluor@ 532, Alexa Fluor@ 546, Alexa Fluor@ 555, Alexa Fluor@ 568, Alexa Fluor@ 594, Alexa Fluor@ 610, Alexa Fluor@ 633, Alexa Fluor@ 647, Alexa Fluor@ 660, Alexa Fluor@ 680, Alexa Fluor@ 700, and Alexa Fluor@ 750), APC, Cascade Blue, Cascade Yellow and Rphycoerythrin (PE), DyLight 405, DyLight 488, DyLight 550, DyLight 650, DyLight 680, DyLight 755, DyLight 800, FITC, Pacific Blue, PerCP, Rhodamine, Texas Red, Cy5, Cy5.5, Cy7, GFP (Green Fluorescent Protein) or derivatives of GFP (e.g., EBFP, EBFP2, Azurite, mKalama1, ECFP, Cerulean, CyPet, YFP, Citrine, Venus, YPet), xanthenes (e.g., rhodamines, rhodols and fluoresceins, and their derivatives); 5-Carboxyfluorescein or 6-Carboxyfluorescein; bimanes; coumarins and their derivatives (e.g. umbelliferone and aminomethyl coumarins); aromatic amines (e.g., dansyl; squarate dyes); benzofurans; fluorescent cyanines; indocarbocyanines; carbazoles; dicyanomethylene pyranes; polymethine; oxabenzanthrane; xanthene; pyrylium; carbostyl; perylene; acridone; quinacridone; rubrene; anthracene; coronene; phenanthrecene; pyrene; butadiene; stilbene; porphyrin; pthalocyanine; lanthanide metal chelate complexes; rare-earth metal chelate complexes; and derivatives of such dyes.

Examples of radionuclides for diagnostic imaging include ^{99m}Tc, ¹¹¹In, ^{68/66}Ga, ¹⁸F, ¹²³I, ⁶⁴Cu, ²¹³Bi, ⁹⁰Y o Lu¹⁷⁷. The radionuclide can be attached to the peptide using a bifunctional chelating agent, such as, but not limited to, mercaptoacetyltriglycine; diaminedithiol; 2-hydrazinonicotinic acid; N-succinimidyl-4-[¹⁸F]fluorobenzoate; 1,4,8,11-tetraazacyclotetradecane-1,4,8,11-tetraacetic acid; N-succinimidyl-3-iodobenzoate; N-succinimidyl-5 -iodo-3 -pyridinecarboxylate; diethylenetriaminepentaacetic acid; 1,4, 7,1 0-tetraazacyclododecane-1,4, 7,1 0-tetraacetic acid; and 1,4, 7 -triazacyclononane-1,4, 7-triacetic acid.

Examples of ferromagnetic compound include Fe, Co or Ni. Examples of paramagnetic compounds include Ferumoxytol.

In the present invention, the above-mentioned lists of examples are also encompassed as preferred embodiments for the conjugate of the invention.

The detectable signal emitted by the detectable agent may be detected and/or measured by any technique known for the skilled person, that include, but are not limited to, fluorescent imaging, computed tomography (CT), magnetic resonance imaging (MRI), optical imaging, single photon emission computed tomography (SPECT), positron emission tomography (PET), x-ray imaging, gamma ray imaging, and the like.

In another preferred embodiment of the conjugate of the invention, alone or in combination with the other preferred embodiments, the peptide of the invention is linked to a therapeutic agent or detectable agent in a proportion 1:1 (peptide of the invention detectable agent or peptide of the invention therapeutic agent) .

In another aspect, the present invention relates to a pharmaceutical composition that comprises the peptide of the invention or the conjugate of the invention, hereinafter "the pharmaceutical composition of the invention".

In the present invention, the terms "pharmaceutical composition" and "pharmaceutical formulation" (or "formulation") are used interchangeably and denote a mixture or solution comprising a therapeutically effective amount or diagnostically effective amount of an active ingredient (i.e the peptide of the invention or the conjugate of the invention) together with one or more pharmaceutically acceptable excipients to be administered to a subject, e.g., a human in need thereof.

Thus, in a preferred embodiment, the pharmaceutical composition of the invention further comprises a pharmaceutically acceptable excipient.

In the present invention the "pharmaceutically acceptable" may refer a material, such as a carrier or diluent, which does not abrogate the biological activity or properties of the compound, and is relatively nontoxic, e.g., the material may be administered to an individual without causing undesirable biological effects or interacting in a deleterious manner with any of the components of the composition in which it is contained.

The terms "pharmaceutically acceptable excipient", "pharmaceutically acceptable carrier", "pharmaceutically acceptable vehicle" and "therapeutically inert excipient" can be used interchangeably and denote any pharmaceutically acceptable ingredient in a pharmaceutical composition having no therapeutic activity and being non-toxic to the subject administered, such as disintegrators, binders, fillers, solvents, buffers, tonicity agents, stabilizers, antioxidants, surfactants, carriers, diluents, excipients, preservatives or lubricants used in formulating pharmaceutical products.

The term "pharmaceutically acceptable salts" denotes salts which are not biologically or otherwise undesirable. Pharmaceutically acceptable salts include both acid and base addition salts. A "pharmaceutically acceptable salt" can refer to a formulation of a compound or agent that does not cause significant irritation to an organism to which it is administered and/or does not abrogate the biological activity and properties of the compound or agent.

The pharmaceutical composition of the invention may comprise an effective amount of the peptide of the invention or the conjugate of the invention.

In the present invention, the term "effective amount" refers to is an amount sufficient to effect beneficial or desired results (e.g. diagnostic of therapeutic effect such as the treatment and/or prevention of a disease). An effective amount can be administered in one or more administrations, applications or dosages. Such delivery is dependent on a number of variables including the time period for which the individual dosage unit is to be used, the bioavailability of the agent, the route of administration, etc.

The peptide, conjugate or pharmaceutical composition of the invention can be formulated to be administered by any suitable route of administration, including oral, parenteral, intraperitoneal, topical, intravenous, inhalation, intramuscular or intranasal routes.

The peptide, conjugate or pharmaceutical composition of the invention can be formulated as a liquid, solid, powder or gel.

According to the experimental results obtained by the present inventors, the peptide and conjugate of the invention may be used for therapeutic purposes.

In another aspect, the present invention relates the peptide, conjugate or pharmaceutical composition of the invention for use as a medicament, hereinafter "the medical use of the invention".

The medical use of the invention may also be formulated as the use of the peptide or the conjugate of the invention for the manufacture of a medicament.

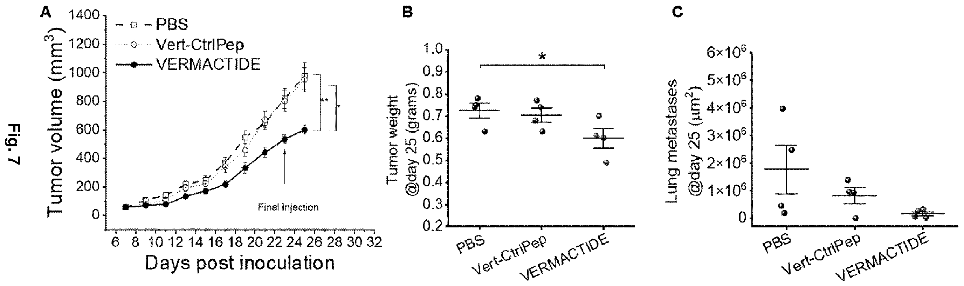

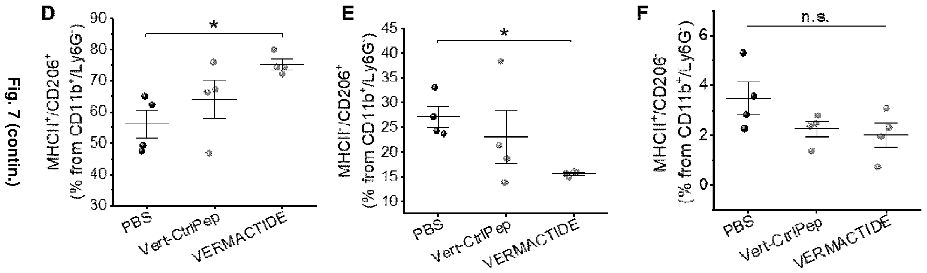

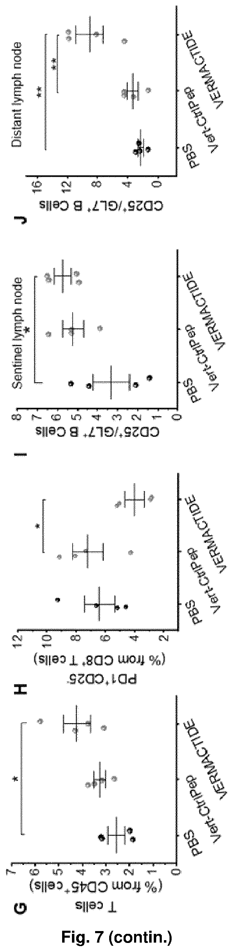

As it was observed in the provided examples, the use of the peptide reduced the tumor progression and metastasis in subjects suffering from cancer (Fig. 7).

Thus, in another aspect, the present invention relates to the peptide of the invention, the conjugate of the invention or the pharmaceutical composition of the invention for use in the treatment and/or prevention of cancer in a subject, hereinafter "the use in cancer of the invention".

Cancer (medical term: malignant neoplasm) is a class of diseases in which a group of cells display uncontrolled growth (division beyond the normal limits), invasion (intrusion on and destruction of adjacent tissues), and sometimes metastasis (spread to other locations in the body via lymph or blood). These three malignant properties of cancers differentiate them from benign tumors, which are self-limited, and do not invade or metastasize. Most cancers form a tumor but some, like leukemia, do not. According to the invention, the terms "cancer" and "tumor" or "cancer disease" and "tumor disease" are generally used interchangeably herein to refer to diseases wherein cells display an uncontrolled growth and optionally invasion and/or metastasis. Preferably, a "cancer" according to the invention is characterized by comprising cells expressing CD206, more preferably macrophages expressing CD206 on their surface, even more preferably wherein the macrophages expressing CD206 on their surface are M2-macrophages, wherein said macrophages are comprise in the tumor microenvironment (tumor-associated macrophages).

In a preferred embodiment of the use in cancer of the invention, alone or in combination with the other preferred embodiments, the cancer is selected from the list consisting of breast cancer, medulloblastoma, melanoma, hepatocellular carcinoma, lung cancer, prostate cancer, bladder cancer, ovarian cancer, leukemia, lymphoma, renal carcinoma, pancreatic cancer, epithelial carcinoma, gastric cancer, colon carcinoma, duodenal cancer, pancreatic adenocarcinoma, mesothelioma, glioblastoma multiforme, astrocytoma, multiple myeloma, prostate carcinoma, hepatocellular carcinoma, cholangiosarcoma, pancreatic adenocarcinoma, head and neck squamous cell carcinoma, colorectal cancer, intestinal-type gastric adenocarcinoma, cervical squamous-cell carcinoma, osteosarcoma, epithelial ovarian carcinoma, acute lymphoblastic lymphoma, myeloproliferative neoplasms, and sarcoma.

In the present invention the term "subject" refers to mammals, wherein mammals include without limitation, human beings and non-human mammals. Examples of mammals include, but are not limited to, any member of the mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. Preferably, the mammal is a human.

In another preferred embodiment of the use in cancer of the invention, alone or in combination with other preferred embodiments, the subject is a human being or a non-human mammal.

In another preferred embodiment of the use in cancer of the invention, alone or in combination with other preferred embodiments, the peptide, conjugate or pharmaceutical composition of the invention is administered to the subject.

The terms "administer," "administering", "administration," and the like, as used herein, means that the peptide of the invention, the conjugate of the invention or the pharmaceutical composition of the invention is delivered to a subject to enable the contact with the desired site of biological action. The administration may be performed by any suitable administration route of including are not limited to oral routes (p.o.), intraduodenal routes (i.d.), parenteral injection (including intravenous (i.v.), subcutaneous (s.c.), intraperitoneal (i.p.), intramuscular (i.m.), intravascular or infusion (inf.)), topical (top.) and rectal (p.r.) administration. The administration may be periodical administration that may comprise the administration every day, every two days, every three days, every four days, every five days, every six days, every seven days, at least once a week, at least once every two weeks or at least once every three weeks.

In another preferred embodiment of the use in cancer of the invention, alone or in combination with other preferred embodiments, the peptide, conjugate or pharmaceutical composition of the invention is administered to the subject by oral, parenteral, intraperitoneal, topical, intravenous, inhalation, intramuscular or intranasal route; more preferably oral route.

Another preferred embodiment of the use in cancer of the invention, alone or in combination with other preferred embodiments, refers to the conjugate of the invention (or the pharmaceutical composition of the invention comprising the conjugate of the invention) that comprises a therapeutic agent for use in the treatment/and or prevention of cancer; preferably wherein the therapeutic agent is a photosensitizer; more preferably wherein the therapeutic agent is a photosensitizer selected form the list consisting of verteporfin, 5-aminolevulinic acid (5-ALA), chlorin e6, IRDye700DX and Porfimer sodium (Photofrin); preferably verteporfin; more preferably wherein the conjugate has the formula (II).

In another preferred embodiment of the use in cancer of the invention, alone or in combination with other preferred embodiments, the peptide or conjugate (that may be comprised in the pharmaceutical composition of the invention) is administered to the subject at a therapeutically effective amount, preferably wherein the therapeutically effective amount refers to an amount of the peptide or the conjugate of the invention enough for the reduction and/or alleviation of one or more signs, symptoms, or causes of cancer in the subject.

In another preferred the of the use in cancer of the invention, alone or in combination with other preferred embodiments, the subject the administration is previous to a photodynamic therapy; so preferably, the subject undergoes a photodynamic therapy.

In another preferred the of the use in cancer of the invention, alone or in combination with other preferred embodiments, the subject does not undergo a photodynamic therapy.

In another preferred embodiment of the use in cancer of the invention, alone or in combination with other preferred embodiments, the peptide or conjugate (that may be comprised in the pharmaceutical composition of the invention) is administered to the subject at a therapeutically effective amount between 0.1 mg/kg to 100 mg/kg; preferably the therapeutically effective amount is 0.1 mg/kg, 0.5 mg/kg, 1 mg/kg, 3 mg/kg, 5 mg/kg, 10 mg/kg, 15 mg/kg, 20 mg/kg, 25 mg/kg, 30 mg/kg, 35 mg/kg, 40 mg/kg, 45 mg/kg, 50 mg/kg, 60 mg/kg, 70 mg/kg, 80 mg/kg, 90 mg/kg or 100 mg/kg; wherein mg/kg refers to the milligrams of the peptide of the invention or the conjugate of the invention with respect to the bodyweight in kilograms of the subject.

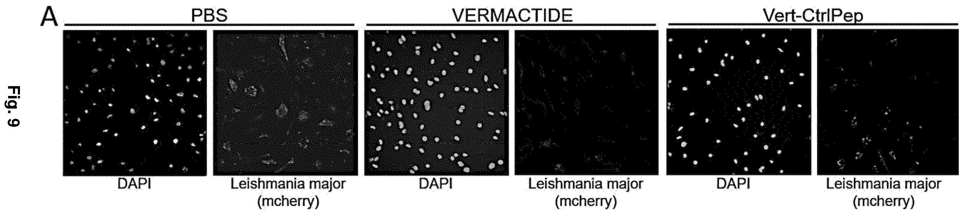

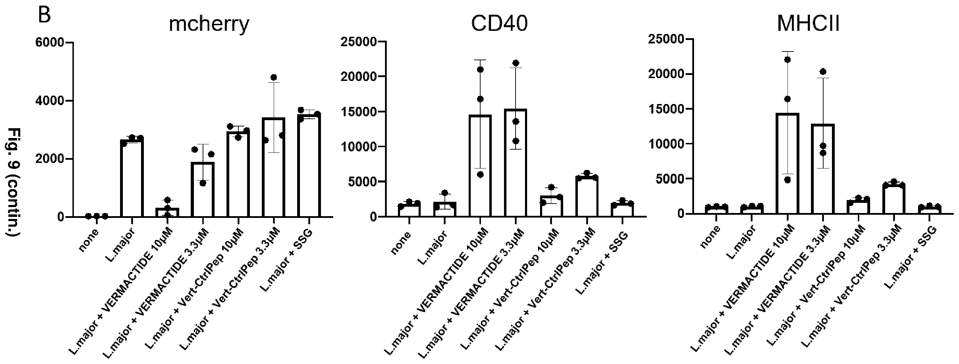

Moreover, phenotype M2 of macrophages is also implicated in the progression of other types of diseases such as infections caused by a protozoon. Surprisingly, the present inventors also found effective the use of the peptide of the invention against a protozoon belonging to *Lehismania* (Fig. 9).

Thus, in another aspect, the present invention relates to the peptide of the invention, the conjugate of the invention or the pharmaceutical composition of the invention for use in the treatment and/or prevention of an infection caused by a protozoon belonging to the genus *Leishmania,* hereinafter "the anti-leishmanial use of the invention".

As used herein, the term "infection" refers to the pathogenic invasion and/or expansion of pathogenic organisms, such as protozoa, in a subject. The infections caused by protozoa belonging to *Leishmania* cause chills, cold sweats, swollen lymph nodes, swollen abdomen (belly) from an enlarged spleen, weight loss, fatigue, weakness, dark or discoloured patches of skin. The infection caused by a protozoon belonging to *Leishmania* is defined as Leishmaniosis.

In another preferred embodiment of the anti-leishmanial use of the invention of the invention, alone or in combination with other preferred embodiments, the protozoon belonging to *Leishmania* is selected form the list consisting of *Leishmania major, Leishmania aethiopica, Leishmania braziliensis* and *Leishmania donovani;* preferably *Leishmania major.*

In another preferred embodiment of the anti-leishmanial of the invention, alone or in combination with other preferred embodiments, the subject is a human being or a non-human mammal.

In another preferred embodiment of the anti-leishmanial of the invention, alone or in combination with other preferred embodiments, the peptide, conjugate or pharmaceutical composition of the invention is administered to the subject.

In another preferred embodiment of the anti-leishmanial of the invention, alone or in combination with other preferred embodiments, the peptide, conjugate or pharmaceutical composition of the invention is administered to the subject by oral, parenteral, intraperitoneal, topical, intravenous, inhalation, intramuscular or intranasal route; more preferably oral route.

Another preferred embodiment, alone or in combination with other preferred embodiments, refers to the conjugate of the invention (or the pharmaceutical composition of the invention comprising the conjugate of the invention) that comprises a therapeutic agent for the treatment and/or prevention of an infection caused by a protozoon belonging to the genus *Leishmania;* preferably wherein the therapeutic agent is a photosensitizer; more preferably wherein the therapeutic agent is a photosensitizer selected form the list consisting of verteporfin, 5-aminolevulinic acid (5-ALA), chlorin e6, IRDye700DX and Porfimer sodium (Photofrin); preferably verteporfin; more preferably wherein the conjugate has the formula (II).

In another preferred embodiment of the anti-leishmanial of the invention, alone or in combination with other preferred embodiments, the peptide or conjugate (that may be comprised in the pharmaceutical composition of the invention) is administered to the subject at a therapeutically effective amount, preferably wherein the therapeutically effective amount refers to an amount of the peptide or the conjugate of the invention enough to reduce and/or alleviate one or more signs, symptoms, or causes of Leishmaniosis in the subject.

In another preferred embodiment of the anti-leishmanial use of the invention of the invention, alone or in combination with other preferred embodiments, the peptide or conjugate (that may be comprised in the pharmaceutical composition of the invention) is administered to the subject at a therapeutically effective amount between 0.1 mg/kg to 100 mg/kg; preferably the therapeutically effective amount is 0.1 mg/kg, 0.5 mg/kg, 1 mg/kg, 3 mg/kg, 5 mg/kg, 10 mg/kg, 15 mg/kg, 20 mg/kg, 25 mg/kg, 30 mg/kg, 35 mg/kg, 40 mg/kg, 45 mg/kg, 50 mg/kg, 60 mg/kg, 70 mg/kg, 80 mg/kg, 90 mg/kg or 100 mg/kg; wherein mg/kg refers to the milligrams of the peptide of the invention or the conjugate of the invention with respect to the bodyweight in kilograms of the subject.

In the present invention, the terms "treat," "treating" or "treatment," as used herein, include alleviating, abating or ameliorating at least one symptom of a disease or condition, preventing additional symptoms, inhibiting the disease or condition, e.g., arresting the development of the disease or condition, relieving the disease or condition, causing regression of the disease or condition, relieving a condition caused by the disease or condition, or stopping the symptoms of the disease or condition either prophylactically and/or therapeutically (e.g. the condition is cancer or an infection caused by a protozoon belonging to Leishmania).

In the present invention the "preventing" or "prevention" of a disease state denotes causing the clinical symptoms of the disease state not to develop in a subject that can be exposed to or predisposed to the disease state but does not yet experience or display symptoms of the disease state.

Moreover, the present inventors tested the ability of the peptide of the invention linked to a detectable agent for use in imaging methods that allow to detect cancer or tumoral tissues in a subject, *in vivo.* Since the peptide of the invention specifically targets cells expressing CD206 on their surface, such as M2 macrophages present in the tumoral microenvironment, said peptide is able to target cancerous or tumoral tissues. Thus, when the peptide of the invention is linked to a detectable agent and is administered to a subject, the detectable signal emitted by said agent can be used for detecting, diagnosing and/or monitoring cancer in the subject.

Therefore, another aspect of the invention relates to peptide of the invention, the conjugate of the invention or the pharmaceutical of the invention for use in the detection, diagnosis and/or monitoring of cancer in a subject, hereinafter "the diagnostic use of the invention".

In the present invention the term "detection" or "detecting" refers to the method for determining if a subject comprises tumoral or cancerous tissue in his/her body, more specifically in some of her/his tissue(s) or organ(s), preferably the detection may be carried out in a subject who has been already diagnosed with cancer and said detection allows to determine the location of the cancerous or tumoral tissues and cells.

In the present invention the term "diagnosis" or "diagnosing" refers to the method for establishing if a subject, suspected from suffering from cancer, suffers from cancer or not. The diagnosis can be established also in combination with other clinical parameters of the subject.

In the present invention, the term "monitor" or "monitoring" refers to the method for the refers to predicting and/or assessing the course of such disease in a subject. Monitoring the evolution of a disease, in the present invention the disease cancer, can facilitate clinical decisions, such as selecting an appropriate treatment, defining groups of patients according to the risk of progression or regression of the disease of interest, or improving the design and analysis of clinical studies and trials by stratifying patients.

In another preferred embodiment of diagnostic use of the invention, alone or in combination with the other preferred embodiments, the peptide, conjugate or pharmaceutical composition of the invention is administered to the subject.

In another preferred embodiment of the diagnostic use of the invention, alone or in combination with the other preferred embodiments, the peptide, conjugate or pharmaceutical composition of the invention is administered to the subject by oral, parenteral, intraperitoneal, topical, intravenous, inhalation, intramuscular or intranasal route; more preferably oral route.

Another preferred embodiment of the diagnostic use of the invention, alone or in combination with the other preferred embodiments, refers to the conjugate of the invention (or the pharmaceutical composition of the invention comprising the conjugate of the invention) that comprises a detectable agent for use in the detection, diagnosis and/or monitoring of cancer in a subject; preferably the detectable agent is selected from the list consisting of a fluorophore, a radionuclide, an enzyme, a metal, barium sulphate, a ferromagnetic compound, a paramagnetic compound, a diamagnetic compound and any combination thereof.

Once the detectable agent linked to the peptide of the invention reaches its target (cells expressing CD206, such as M2 macrophages of the tumoral microenvironment), the signal emitted by said detectable agent can be measured enhancing the capacity of differentiating the target cell or tissue from other cells or tissues (that do not comprise the target of the peptide of the invention). In the present invention, the terms "measured" or "measuring" are equivalent to quantified or quantifying.

In another preferred embodiment of the diagnostic use of the invention, alone or in combination with the other preferred embodiments, the detection, diagnosis and/or monitoring is carried out *in vivo.*

In another preferred embodiment of the diagnostic use of the invention, alone or in combination with the other preferred embodiments, the peptide, conjugate or pharmaceutical composition of the invention is used as a contrast agent for the detection, diagnosis and/or monitoring of cancer in a subject; preferably *in vivo.*

In another preferred embodiment of the diagnostic use of the invention, alone or in combination with the other preferred embodiments, the subject is a human being or a non-human mammal.

As used in the present invention, the term "contrast agent" refers to a substance or compound used to increase the contrast or enhance the visibility of a target cell, tissue, structure or organ, therefore the contrast agent allows to highlight a specific area of a subject, rendering organs, cells, blood vessels, tissues, and/or other portions to be more detectable and/or more clearly imaged.

The detectable signal emitted by the detectable agent may be detected and/or measured by any technique known for the skilled person, that include, without limitation, fluorescent imaging, computed tomography (CT), magnetic resonance imaging (MRI), optical imaging, single photon emission computed tomography (SPECT), positron emission tomography (PET), x-ray imaging, gamma ray imaging, and the like.

In another preferred embodiment of the diagnostic use of the invention, alone or in combination with other preferred embodiments, the peptide or conjugate (that may be comprised in the pharmaceutical composition of the invention) is administered to the subject at a diagnostically effective amount, preferably wherein the diagnostically effective amount refers to an amount of the peptide or the conjugate of the invention enough for the detection, diagnosis and/or monitoring of cancer in a subject.

In another preferred embodiment of the diagnostic use of the invention, alone or in combination with other preferred embodiments:
i) the level of the detectable signal emitted by the detectable agent is detected and/or measured after the peptide of the invention linked to the detectable agent is administered to the subject;
ii) the level of the detectable signal detected and/or measured in step i) is compared to a reference detectable signal;
wherein a greater level of the detectable signal of step i) compared to the reference detectable signal indicates the presence of cancer or a tumoral tissue in the subject.

In the present invention, the term "reference detectable signal" refers to the level of a detectable signal emitted by a detectable agent, preferably linked to the peptide of the invention, wherein said detectable agent or peptide comprising it has been administered to subject (or group of subjects) who does not suffer from cancer. The reference detectable signal may also refer to the level of a detectable signal emitted by a detectable agent, preferably linked to the peptide of the invention, wherein said detectable agent or peptide comprising it has been administered to a subject, and wherein the detectable signal is detected and/or measured in a tissue, organ or part of the body of a subject that does not comprise cancerous or tumoral tissues or cells related to it.

In another preferred embodiment of the diagnostic use of the invention, alone or in combination with other preferred embodiments:
i) the level of the detectable signal emitted by the detectable agent is detected and/or measured after the peptide of the invention linked to the detectable agent is administered to the subject in at least two different points in time;
wherein an increased level of the detectable signal throughout time indicates that cancer is progressing negatively.

In the present invention, a level is understood to be "greater", "higher", "increased" or "increased" with respect to the levels with which it is compared when it is at least 1.5 times, 2 times, 3 times, 4 times, 5 times, 6 times, 7 times, 8 times, 9 times, 10 times, 20 times, 30 times, 40 times, 50 times, 60 times, 70 times, 80 times, 90 times, 100 times or even more, with respect to the levels with which it is compared.

In the present invention the expression "cancer is progressing negatively", refers to process by which the cancer disease progresses to a more pathogenic stage, meaning that the cancer or cancerous tissues, grow, invade other tissues, organs, metastasize or show resistance to the provided treatments.

In the present invention the term "detectable signal" refers to any signal detectable by any conventional method, wherein the detectable signal may be a signal within the visible spectrum, a fluorescent signal, a radioactive signal or the like.

In the present invention, the use in cancer of the invention may be formulated as:
- A method for treating and/or preventing cancer in a subject, that comprises administering the peptide of the invention, the conjugate of the invention or the pharmaceutical composition of the invention to the subject; or
- Use of the peptide of the invention the conjugate of the invention in the manufacture of a medicament for the treatment and /or prevention of cancer in a subject.

In the present invention, the anti-leishmanial use of the invention may be formulated as:
- A method for treating and/or preventing an infection caused by a protozoon belonging to Leishmania in a subject, that comprises administering the peptide of the invention, the conjugate of the invention or the pharmaceutical composition of the invention to the subject; or
- Use of the peptide of the invention the conjugate of the invention in the manufacture of a medicament for the treatment and /or prevention of an infection caused by a protozoon belonging to Leishmania in a subject.

In the present invention, the diagnostic use of the invention may be formulated as:
- A method for the detection, detection, diagnosis and/or monitoring (preferably in vivo) of cancer in a subject, wherein the method comprises administering the conjugate of the invention linked to a detectable agent to a subject; or
- Use of the peptide of the invention or the conjugate of the invention in the manufacture of a composition for the detection, diagnosis and/or monitoring (preferably in vivo) of cancer in a subject.

In another aspect, the present invention refers to the use of the peptide of the invention or the conjugate of the invention for the *in vitro* detection of CD206, hereinafter "the *in vitro* detection use of the invention".

In a preferred embodiment, alone or in combination with the other preferred embodiments, the *in vitro* detection of the invention refers to the *in vitro* detection of CD206 in a biological sample.

In the present invention, the term "biological sample" refers to a cell, a cell culture, a tissue sample or any biological material, preferably isolated from a subject. The biological sample may contain any biological material suitable for detecting the desired biomarker (i.e. CD206) and may comprise cells and/or non-cellular material from the subject. Preferably, the biological sample may be a cell, a cell culture or a tissue sample isolated from any suitable tissue or biological fluid such as blood, plasma, serum, urine, cerebrospinal fluid (CSF), muscle tissue, tumoral tissue, lung tissue, tissue surrounding a tumor, tissue, lymph tissue, heart or brain.

In a preferred embodiment, alone or in combination with the other preferred embodiments, the *in vitro* detection use of the invention refers to the *in vitro* detection of macrophages that express CD206; preferably wherein the macrophages are isolated, are part of a culture or part of a tissue isolated from a subject.

In a more preferred embodiment, alone or in combination with the other preferred embodiments, the *in vitro* detection use of the invention refers to the *in vitro* detection of macrophages that express CD206, preferably M2 macrophages, in a tissue sample isolated from subject; preferably wherein the tissue sample is a tumoral sample isolated from a subject.

In another aspect, the present invention refers to an *in vitro* method for detecting CD206 in a biological sample, hereinafter "the *in vitro* detection method of the invention", that comprises:
a) contacting the biological sample with the peptide of the invention or the conjugate of the invention, and
b) detecting the presence of the invention or the conjugate of the invention bound to the biological sample.

The step b) of the *in vitro* detection method of the invention may be carried out by any suitable method known for the skilled person; preferably the step b) may be carried out by detecting a detectable signal of a detectable agent that is linked to an antibody, or a fragment thereof, that specifically binds the peptide of the invention or the complex peptide of the invention-CD206; or step b) may be carried out by detecting a detectable signal of the detectable agent comprised in the conjugate of the invention.

In another preferred embodiment, alone or in combination with the other preferred embodiments, the *in vitro* detection method of the invention further comprises a step a') performed before the step b), wherein said step a') comprises:
a") separating the peptide of the invention or conjugate of the invention that is not bound to CD206.

In a preferred embodiment, alone or in combination with the other preferred embodiments, the *in vitro* detection method of the invention refers to the *in vitro* detection of macrophages that express CD206; preferably wherein the macrophages are isolated, are part of a culture or part of a tissue isolated from a subject.

In a more preferred embodiment, alone or in combination with the other preferred embodiments, the *in vitro* detection method of the invention refers to the *in vitro* detection of macrophages that express CD206, preferably M2 macrophages, in a tissue sample isolated from a subject; preferably wherein the tissue sample is a tumoral sample isolated from a subject.

In another aspect, the present invention refers to use *in vitro* of the peptide of the invention or the conjugate of the invention for promoting the conversion of a M2-macrophage to M1-macrophage, hereinafter "the conversion use of the invention".

The expression "conversion of a M2-macrophage to M1-macrophage" refers to the shift of the M2-macrophage phenotype to a M1-macrophage phenotype in a macrophage, preferably promoted by the contact with the peptide or conjugate of the invention.

M1-macrophage phenotype refer to macrophages that show a M1 phenotype characterised in that the macrophage show pro-inflammatory activity (i.e secrete pro-inflammatory cytokines), and wherein said macrophages express major histocompatibility complex II molecules (MHCII+) and do not express CD206 (CD206-).

M2-macrophage phenotype refer to M2-macrophages that show anti-inflammatory activity (i.e secrete anti-inflammatory cytokines), and wherein said macrophages do not express MHC-II molecules (MHCII-) but they express CD206 (CD206+).

The conversion of a M2-macrophage phenotype to M1-macrophage phenotype may be total or partial, wherein the partial conversion refers to the conversion M2-macrophages to an intermediate phenotype M1/M2. The M1/M2 phenotype refers to macrophages that show characteristics of both M1 and M2 macrophages, preferably M1/M2 macrophages express major histocompatibility complex II molecules (MHCII+) and CD206 (CD206+).

The characteristics of M1 and M2 macrophage phenotypes may be determined as in the examples 6, 7 and 8.

In another aspect, the present invention refers to an *in vitro* method for promoting the conversion of a M2-macrophage into M1-macrophage, hereinafter "the *in vitro* conversion method of the invention" that comprises:
a) contacting the peptide of the invention or the conjugate of the invention with the M2-macrophage or a biological sample comprising the M2-macrophage.

In a preferred embodiment, the *in vitro* conversion method of the invention, alone or in combination with the other preferred embodiments, further comprises a step b) of incubating after step a), preferably wherein the incubation under suitable conditions that allow the peptide or conjugate of the invention to bind to the CD206 comprised or expressed in a macrophage (preferably the M2-macrophage) and therefore, the conversion of the M2-macrophage to the M1-macrophage. The suitable conditions for incubation may be determined routinely by the skilled person.

### DESCRIPTION OF THE DRAWINGS

**Fig. 1****. Design and molecular dynamics of MACTIDE. A: Design of MACTIDE based on Sunflower Trypsin Inhibitor I (SFTI-1) and the CD206-binding motif mUNO** . FAM-Ahx (represents the Fluorescein-aminohexanoic acid linker attached to the N-terminus. B: Root mean square deviations (RMSD) for heavy atoms of mUNO (gray line) and MACTIDE (black line), in two 400 ns molecular dynamics for mUNO and MACTIDE in solution indicating the two most populated conformations of MACTIDE, C0 and C1, structures of the C0 and C1 shown on the right. C: Root mean square fluctuation (RMSF) of each residue for MACTIDE in solution. D: Hydrogen bonds in MACTIDE (dashed gray line), between Pro7-Cys10 with an occupancy of 75 % and between Ile6-Thr3 with an occupancy of 10% (present in the C1 conformation).

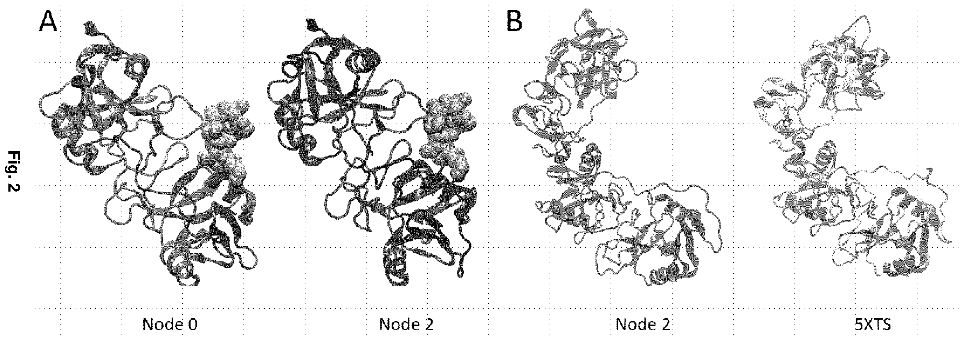

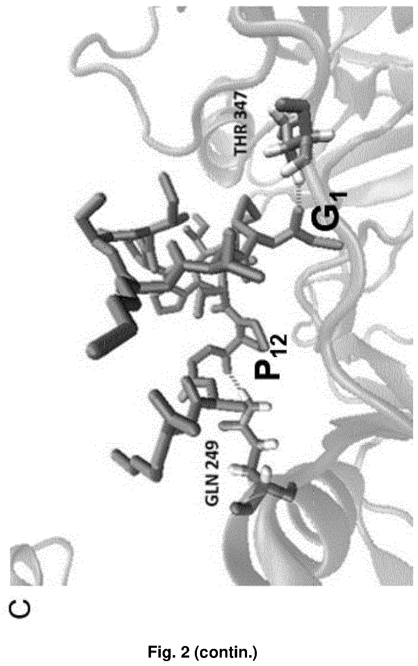

**Fig. 2****. Docking shows high binding energy of MACTIDE to CD206 and ligand-induced conformational change. A:** CTLD2 domain for cluster node 0 (left) compared with cluster node 2 (right). MACTIDE is represented as VdW spheres. A large alpha helix displacement of 3.8 Å was found in node 2 making space for the peptide to bind. **B:** node 2 (left) compared with the crystal structure 5XTS(right). Significant differences were found at the CysR domain. **C:** Hydrogen bonds formed at the docking pose.
**Fig. 3****. MACTIDE has higher affinity and proteolytic stability than mUNO.** A: QCM experiment of MACTIDE and mUNO and control peptides at final concentration of 10µM in PBS on multilayers of PAH/CD206 or control multilayers of PAH/BSA. The black arrow indicates when the peptides were added, gray arrows indicate when the washing step with PBS were started. B: Integrity of FAM-MACTIDE and FAM-mUNO measured by LC-MS at different time points after incubation of peptides with lysate derived from a 4T1 tumor.

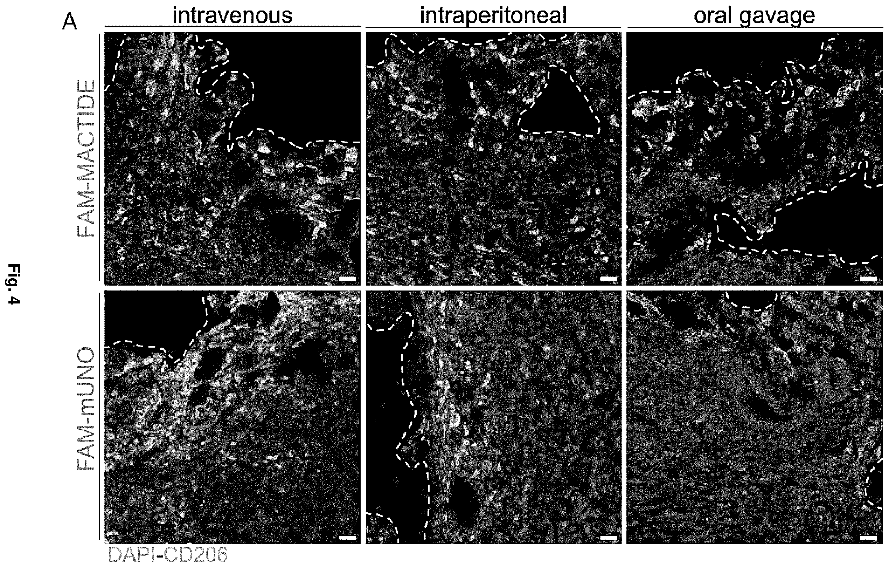

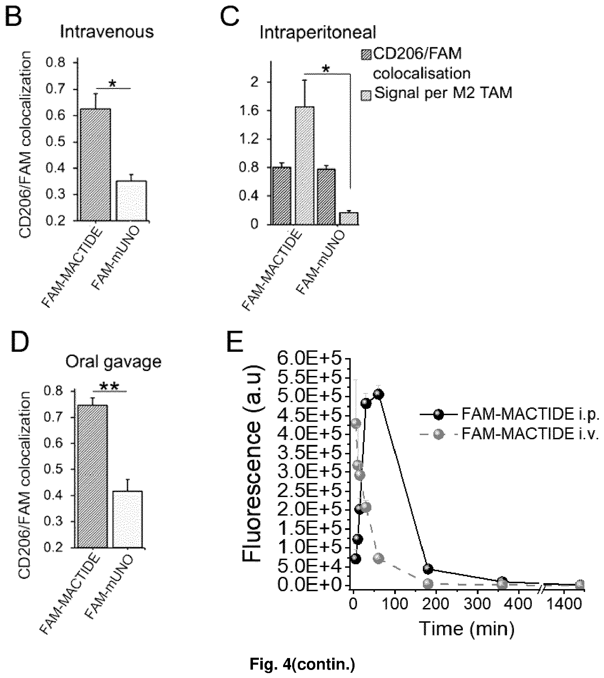

**Fig. 4****. FAM-MACTIDE targets CD206+ TAM using different administration routes.** Thirty nmoles of FAM-MACTIDE or FAM-mUNO were administered i.p., i.v. or orally and left to circulate for 24 h. At 24 h, the mice were sacrificed, and the organs were collected, fixed, cryoprotected, sectioned, and immunostained for FAM and CD206 (colocalization can be seen in the images as brigh areas) **(A).** The CD206/FAM colocalization indices were calculated from representative images from N=3 tumors using imaged (Mandler's tM2 index) **(B, C, D)** and the signal intensity per number of CD206+ TAMs was quantified using imaged for the i.p. administration **(C). (E):** Pharmacokinetics of i.p. and i.v. administered FAM-MACTIDE (30 nmoles) analyzed by detecting plasma fluorescence in the FAM channel at different timepoints (N=3). Scale bars represent 20 µm. **P* ≤ 0.05, ***P* ≤ 0.01.

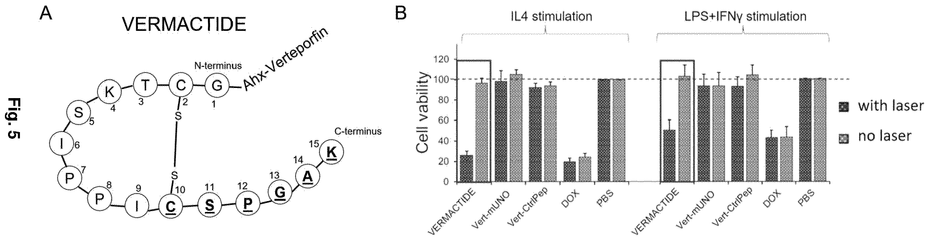

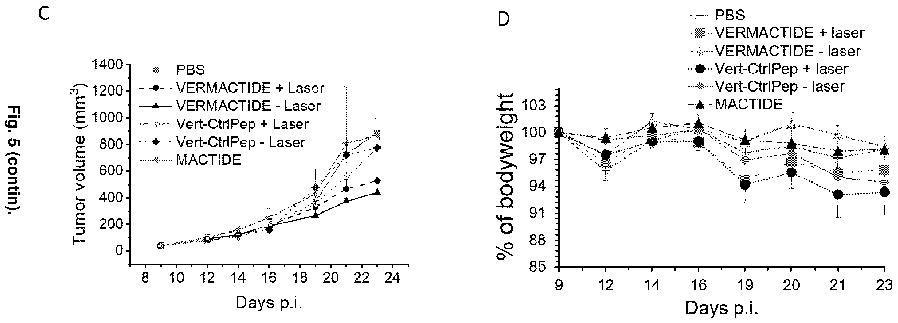

**Fig. 5****. In vitro and in vivo photodynamic therapy with VERMACTIDE. A:** Structure of VERMACTIDE. **B:** VERMACTIDE, Vert-CtrlPep and DOX (doxorubicin) were incubated with primary human M2 macrophages (obtained from monocytes derived from human blood) at 30µM for one hour at 37°C, followed by 2 washes with medium. Then, the groups shown in red were irradiated with 10 J/cm² (irradiance: of 170 mW/cm², spot diameter: 0.5 cm). Then, the cells were incubated for 48 hrs. and the cell viability was determined using the MTT((3-(4,5-Dimethylthiazol-2-yl)-2,5-Diphenyltetrazolium Bromide) assay. **C:** Treatment with VERMACTIDE and Vert-CtrlPep with and without laser (denoted by + and - respectively), in mice bearing orthotopic 4T1 tumors (N=6) in Balb/C mice by injecting 20×10⁴ 4T1 cells in the mammary fat pad. Conjugates were administered intraperitoneally when tumors reached 30 mm³ and were performed every 4-5 days to give a total of 4 injections. The dose of Vert-Peptide conjugates was 30nmoles (1 mg/Kg in Vert). For the irradiated groups, 6 hours after administration of conjugates or PBS, mice were irradiated with 100 J/cm² (irradiance: of 170 mW/cm², spot diameter: 0.5 cm), shown is primary tumor volume progression during treatment. Treatments were administered on days 9, 16, 20, 22 p.i... **D:** Bodyweight during the treatment as % of their initial bodyweight.

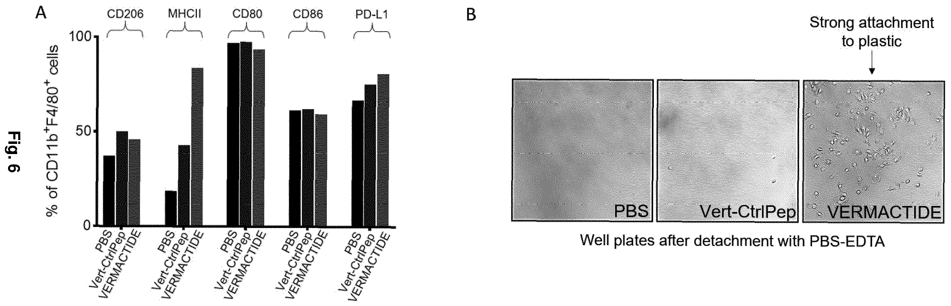

**Fig. 6****. VERMACTIDE increases MHCII and macrophage attachment.** Day 5 BMDM were incubated during 4h with 10µM conjugates, washed and followed-up for 48hr, and characterized with cytometry **(A).** After the in vitro treatment, macrophages treated with VERMACTIDE were found to strongly adhere to the well plate **(B).**
**Fig. 7****. VERMACTIDE slows tumor growth, metastasis and induces a mixed M1/M2 phenotype.** 4T1.2 bearing mice were treated with 9 doses i.p. every other day of Vert-CtrIPep, VERMACTIDE (1mg/Kg in Verteporfin per dose) or PBS. The primary tumor volume was monitored **(A),** and the mice were then sacrificed, and their tumor weights analyzed **(B)** and pulmonary metastases area quantified from H&E sections **(C)** and their tumors analyzed with flow cytometry **(D-J).**

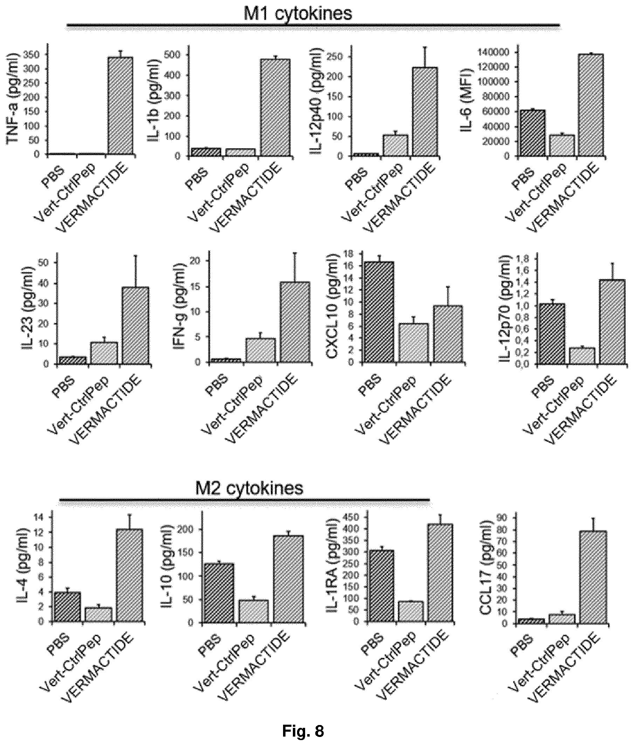

**Fig. 8****. Effect of VERMACTIDE during monocyte-macrophage transition.** Monocytes derived from PMBCs were treated with M-CSF together with Vert-CtrlPep, VERMACTIDE or PBS (30µM for 72 hrs.) and the level of cytokines in the supernatant was evaluated 72hrs later using LegendPlex.
**Fig. 9****. Anti-leishmanial activity of VERMACTIDE.** A) confocal microscopy of L. major - infected macrophages, treated with VERMACTIDE Vert-CtrlPep or left untrated (PBS). B) Flow cytometry fluorescence plots for different markers in macrophages: mcherry (for staining of macrophages infected with L. major), CD40 and MHCII. The y-axis represents the mean fluorescence intensity.

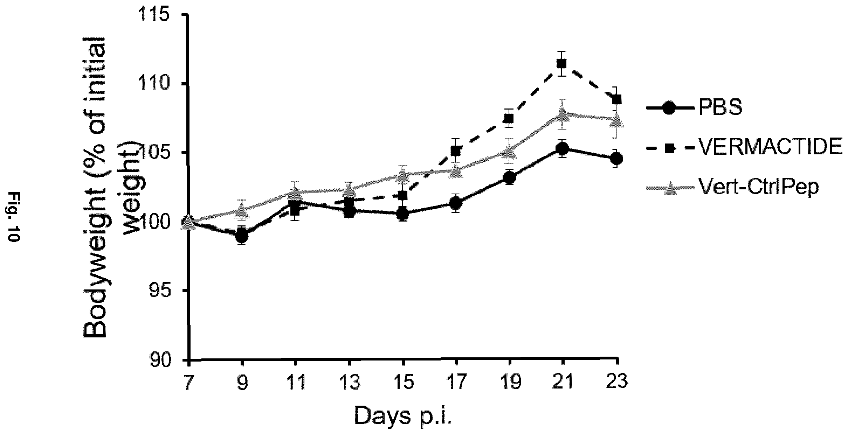

**Fig. 10****. Bodyweights for treatment study of** **figure 7****.**

### Examples

The following examples are provided by the inventors to better illustrate the claimed invention and are not to be interpreted as limiting the scope of the invention.

### Example 1. Design and molecular dynamics of MACTIDE

In our design we removed the cyclization loop of SFTI-1 (SEQ ID NO: 4: GRCTKSIPPICFPD) and in its place inserted mUNO (SEQ ID NO: 3: CSPGAK), we also removed the head-to-tail cyclization to accommodate a fluorophore/drug on the N-terminus. Additionally, we sought to have a flexible spacer between the cyclization loop and the fluorophore/drug so the theranostic moiety is free to move away and not interfere with the binding site on the receptor. To this end we added glycine G1 (Fig. 1A) as glycine has been found to be the amino acid with the highest flexibility. The resulting peptide was named as MACTIDE (SEQ ID NO: 2: GCTKSIPPICSPGAK).

To assess if our modifications constrained the mUNO motif in MACTIDE, we performed computational analyses to study MACTIDE structure in solution. MACTIDE was solvated with water and two independent molecular dynamics simulations were performed, each 400 ns long, to construct an ensemble of configurations. The ensemble was clustered in sub-ensembles using an RMSD criteria, grouping conformations with similar structural arrangements. MACTIDE adopted mainly two configurations, C0 and C1, with variations of about 1Å compared with the average structure (Fig. 1B black). On the other hand, mUNO varied continuously during simulations, with variations reaching 3Å from its average structure (Fig. 1B gray), indicating that MACTIDE is significantly more constrained than mUNO. Both C0 and C1 MACTIDE conformations were found in 44% of the whole trajectory and their structures are shown in Fig. 1B.

We also analyzed the rigidity of MACTIDE through the root mean square fluctuation of each residue (Fig. 1C). Only the two terminals exhibit more flexibility, while the rest of the peptide had similar RMSF values. We then calculated intra hydrogen bonds in MACTIDE (Fig. 1D). There was a moderate hydrogen bond (mainly electrostatic) between Cys10-Pro7, with a high occupancy of 75%. In the C1 conformation, there was an additional hydrogen bond between Ile6-Thr3. Interestingly, the primary loop of the acyclic SFTI-1 only has one hydrogen bond (Thr3-Ile9)¹⁷, and it is not observed in our structure, indicating that the structure of the primary loop in MACTIDE has different characteristics than the structure of the primary loop in acyclic SFTI-1.

### Example 2. Docking reveals high binding energy of MACTIDE to CD206 and ligand-induced conformational change

In order to estimate the binding site of MACTIDE, we used a hierarchical algorithm for blind and flexible peptide docking, HPEPDOCK21. Flexibility of CD206 was treated by a previous simulation of the receptor in solution, followed by a clustering procedure to find the most populated conformations (see Material and Methods). The best docking score was found for a configuration of the receptor in which the alpha helix at CTLD2, defined by Thr360 and Tyr373, is displaced downwards 3.8 Å, generating space for the peptide to accommodate (Fig. 2A). In the peptide-bound configuration, we observed slight differences in the CysR region and a closing of the V-shaped portion of the receptor, respect to the crystal structure 5XTS, (Figure 3B); of note, we observed a similar ligand-induced conformational change in CD206 when bound to mUNO. The docking pose located in the region between lectin domains CTLD1-2, interestingly the same region that binds mUNO, and the peptide made closest contact with Asp273 and Thr324. Hydrogen bonds formed between G1-Thr347 and between P12-Gln249 (Fig. 2C), and the mUNO motif (C10-K15) pointed to the receptor. The HPepDock docking score of MACTIDE was considerably higher than the mUNO docking score against the same receptor: - 181.7 vs -115. Additionally, as G1 participated in a H-bond with the receptor, we performed docking without it and found a lower docking score of -172.5, indicating that G1, besides serving as a flexible spacer, contributes to binding. Based on this data, we decided to synthesize MACTIDE and evaluate it experimentally.

### Example 3. MACTIDE shows enhanced affinity and proteolytic stability

To confirm the capacity of MACTIDE to bind to CD206, we evaluated binding to recombinant CD206 included in a viscoelastic film, using Quartz Crystal Microbalance (QCM). QCM is a powerful technique used to study label-free ligand-receptor interactions in solution (Baltus, R. E. et al., Langmuir 2007, 23 (7), 3880-3885), wherein peptide binding and dissociation is sensed through a mass increase or decrease on a resonating crystal surface functionalized with the receptor. Here, we evaluated the mass changes of MACTIDE when binding and dissociating in solution to CD206 immobilized on a viscoelastic film, deposited on a gold-modified quartz crystal resonating at 5 MHz. Recombinant CD206 was immobilized using layer-by-layer (LBL) self-assembly, an electrostatically-driven adsorption of charged polyelectrolytes on a layer of polyallylamine (PAH) (Flexer, V., et al., Anal. Chem. 2006, 78 (2), 399-407). These experiments showed that MACTIDE bound to PAH/CD206 (Fig. 3A,) and incompletely and slowly dissociated upon washing with PBS (Fig. 3A, gray arrow), whereas two control peptides did not bind to the same multilayer (Fig. 3A,). Additionally, MACTIDE did not bind to the control multilayer PAH/BSA (Fig. 3A,). The mUNO peptide also showed binding to the PAH/CD206 multilayer but with a complete and faster dissociation kinetics than MACTIDE upon washing with PBS (Fig. 3A), whereas the scrambled mUNO peptide did not bind to the same multilayer (Fig. 3A,), and mUNO did not bind to the control multilayer PAH/BSA (Fig. 3A). Fitting the association and dissociation curves revealed that the constant of association was slightly faster for mUNO (6.5 × 10² M⁻¹ s⁻¹ versus 14 × 10² M⁻¹ s⁻¹), but the main difference was in the dissociation constant, which was 30-fold slower for MACTIDE (2.5 × 10⁻⁴ s⁻¹ versus 8.7 × 10⁻³ s⁻¹), resulting in K_{D} = 0.38 µM for MACTIDE and K_{D} = 6 µM for mUNO.

We next evaluated the stability of FAM-MACTIDE against tumor proteases from breast tumors. To this end we incubated both FAM-MACTIDE and FAM-mUNO with a tumor lysate obtained from 4T1 orthotopic breast tumor during different timepoints and evaluated the integrity of the peptide using LC-MS mass spectrometry. These results showed that the half-life of FAM-MACTIDE in this tumor lysate increased 5-fold respect to FAM-mUNO (Fig. 3B).

### Example 4. FAM-MACTIDE targets CD206+ TAM using different administration routes

To show that MACTIDE can be used to deliver a conjugated payload to CD206+ TAMs, we administered fluorescein (FAM)-MACTIDE using different administration routes to 4T1 breast tumor-bearing mice, a model which highly overexpresses CD206 in the tumor macrophages as we previously showed. The targeting efficacy was evaluated by immunostaining tumor sections for FAM and CD206, twenty-four hours after peptide administration.

FAM-MACTIDE showed CD206+ TAM targeting when administered intravenously, whereas low CD206+ TAM targeting was observed for FAM-mUNO using this route (Fig. 4A left, and 4B). With the i.p. administration, both FAM-MACTIDE and FAM-mUNO showed high CD206/FAM colocalization (80%) (Fig. 4A middle, and 4C), but FAM-MACTIDE showed a 10-fold higher FAM intensity per CD206+ TAM (Fig. 4C). Interestingly, FAM-MACTIDE also targeted CD206+ TAMs when administered orally (Fig. 4A right, and 4D). We also determined the blood half-life of FAM-MACTIDE using i.v. and i.p. administration (Fig. 4E). As these values are the same as those obtained previously for FAM-mUNO, we propose that the higher affinity and proteolytic stability of MACTIDE account for the homing differences observed between the two peptides.

### Example 5. VERMACTIDE photodynamic therapy

We next coupled MACTIDE to the photosensitizer Verteporfin, with the aim of depleting CD206+ TAMs using photodynamic therapy. To this end, carboxy Verteporfin was coupled to the N-terminus of MACTIDE, a construct we refer to as "VERMACTIDE" (Fig. 5A). We performed in vitro photodynamic therapy with VERMACTIDE on primary human macrophages stimulated with IL4 or (LPS + IFNγ). Irradiated VERMACTIDE killed 80% of IL4-stimulated macrophages and 40% of (LPS + IFNy)-stimulated macrophages (we previously showed these express CD206 at lower levels); non irradiated VERMACTIDE showed no toxicity to these two cell types. Doxorubicin showed similar toxicity, but was independent of the irradiation, as expected. The control conjugates Vert-mUNO and Vert-CtrlPep showed no toxicity in any of the cells regardless of irradiation (Fig. 5B). Based on these results showing laser-induced and preferential toxicity to IL4-stimulated macrophages, we decided to evaluate the in vivo therapeutic efficacy of irradiated VERMACTIDE in the orthotopic 4T1 model. Four administrations of VERMACTIDE followed by tumor irradiation produced a significant slowing down of volume growth compared to the control groups PBS(+ laser), Vert-CtrIPep(- laser), Vert-CtrIPep(+ laser) and MACTIDE(-laser). Interestingly, non-irradiated VERMACTIDE produced the highest tumor reduction of all groups (Fig. 5C) and the smallest effect on body weight. Based on this, we decided to focus subsequent studies on the inherent effect of VERMACTIDE, speculating that the observed in vivo effect could be mediated by a TAM modulation elicited by Verteporfin, a known inhibitor of the co-transcription factor YAP (Yes Associated Protein).

### Example 6. VERMACTIDE increases MHCII and attachment in mouse macrophages

We treated bone marrow derived macrophages (BMDMs) in vitro with VERMACTIDE or Vert-CtrlPep and evaluated changes in cell surface markers 48 h later. Cytometry revealed significant changes in the expression of MHCII and no changes in CD80, CD86, CD206 or PDL1 (Fig. 6A). After the in vitro treatment, macrophages treated with VERMACTIDE were found to strongly adhere to the well plate (Fig. 6B).

### Example 7. VERMACTIDE increases the MHCII⁺/CD206⁺ TAM population and has therapeutic effect in orthotopic 4T1.2

We evaluated the therapeutic effect of VERMACTIDE on tumor progression and metastasis, using the highly metastatic orthotopic 4T1.2 model. When tumors reached 50mm³ we began dosing with VERMACTIDE or Vert-CtrlPep every other day, until day 23 using the same dose used previously in section 5. VERMACTIDE treated mice showed significantly smaller tumor volumes at day 25, compared to Vert-CtrlPep and PBS (Fig. 7A) and the VERMACTIDE group showed smaller tumor weights (Fig. 7B) and less lungs metastasis (Fig. 7C). No bodyweight loss was detected in any of the groups (Fig. 10). We then analyzed the cell populations in tumor and lymph node using cytometry. VERMACTIDE increased by 20% the MHCII+/CD206+ TAM/monocyte population (Fig. 7E), decreased the M2 population by more than 10% (Fig. 7F) and did not significantly alter the M1 population (Fig 7G). VERMACTIDE duplicated the proportion of T cells in the tumor (Fig. 7H) and respect to Vert-CtrlPep decreased the PD1+CD25- subpopulation of CD8 T cells (Fig. 7I), possibly an immunosuppressive CD8 T cell subpopulation. In both the inguinal lymph nodes, an increase in CD25+ follicular B cells was detected in the VERMACTIDE group (Fig. 7J-K).

### Example 8. VERMACTIDE increases production of M1 cytokines in human macrophages

We evaluated whether VERMACTIDE could have effect on the cumulative cytokine production of human monocytes transitioning to macrophages. After 72 hrs. of in vitro treatment, VERMACTIDE dramatically increased the levels of M1 cytokines TNF-α, IL-1β, IL-12p40 and IL-6, and to a lesser extent those of IL-23 and IFNy, whereas it decreased the levels of CXCL10 respect to PBS. With respect to PBS, VERMACTIDE elicited an appreciable increase in CCL17, and modest increases in the M2 cytokines IL-4, IL-10, IL-1RA (Fig. 8).

### Example 9. Anti-leishmanial activity of VERMACTIDE

Bone marrow derived macrophages (BMDM) were obtained from 7 weeks old female BL6 mice by culturing bone marrow cells in complete RPMI medium containing 30% L929 supernatant during 5 days in sterile, but not tissue-culture treated, 10cm Petri dishes. BMDM were then seeded at 300.000 per well in a 24 well plate containing a sterile coverslip **(****Fig.9A****),** or 100.000 per well in a 96 well plate for cytometry **(****Fig.9B****).** 16h later, macrophages were infected with *Leishmania major* mcherry ("L. major" at a moi of 5). 24h later (day 7), supernatant was removed and replaced with RPMI medium containing VERMACTIDE or Vert-CtrlPep (labelled as "control") at 10 µM or 3.3 µM, or Sodium stibogluconate (labelled as "SSG") at 10µM. Cells were harvested at day 10 and characterized with confocal microscopy **(****Fig.9A****),** using the 10µM conjugate condition) and flow cytometry **(****Fig.9B****)** in the mcherry channel plus staining for markers CD40 and MHCII. Experiments were done at the Salvador Iborra Lab (Madrid, Spain).

### MATERIALS

### Peptides and peptide conjugates

Peptides were synthesized on solid phase. FAM and Vert denote carboxyfluorescein and Verteporfin respectively and they were coupled to the N-terminus of peptides via their carboxylic acid, spaced via an aminohexaonic acid linker (Ahx). Peptides and peptide conjugates were purchased from Lifetein LLC, TAG Copenhagen or synthesized at the peptide synthesis core facility of CNB-CSIC. Peptide sequences can be seen in the following table 1.

**Table 1. Amino acid peptide sequences.**

| **Notation used** | **N-terminus modification** | **C-terminus modification** | **Peptide (N-term to C-term)** | **Notes** |
|---|---|---|---|---|
| FAM-MACTIDE | FAM-Ahx-/ | | GCTKSIPPICSPGAK (SEQ ID NO: 2) | Disulfide : C2-C10 |
| MACTIDE | | | GCTKSIPPICSPGAK (SEQ ID NO: 2) | Disulfide : C2-C10 |
| mUNO | | | CSPGAK (SEQ ID NO: 3) | |
| iRGD | Ac-/ | /-NH₂ | CRGDKGPDC (SEQ ID NO: 6) | Disulfide : C1-C9 |
| CES | Ac-/ | /-NH₂ | CESPLLSEC (SEQ ID NO: 7) | Disulfide : C1-C9 |
| Vert-CtrlPep | Vert-Ahx-/ | | AKPCGS (SEQ ID NO: 8) | |
| VERMACTIDE | Vert-Ahx-/ | | GCTKSIPPICSPGAK (SEQ ID NO: 2) | Disulfide : C2-C10 |

| | | | | |
|---|---|---|---|---|
| **FAM: Carboxyfluorescein; vert: Verteporfin; ahx: aminohexanoic acid; AC: acetyl group; "/" represents how the modification is linked to the peptide. | | | | |

### Docking

Docking was performed after a global sampling of binding orientations along the receptor surface, using HPEPDOCK. In this algorithm, peptide flexibility is accomplished by generating an ensemble of conformations. Then, the generated conformations are globally docked against the entire protein. As receptor flexibility is not contemplated with this method, to address this aspect, we generated an ensemble of receptor conformations from a molecular dynamic trajectory and clustered the total number of configurations with an rmsd criteria. The five most populated configurations for the receptor were selected and used as coordinates for the receptor in each docking calculation.

### Peptide stability in tumor

For peptide stability measurements 200 µl freshly prepared tumor lysate was mixed with 50 µl PBS (as control), 30 µM FAM-mUNO, or 30 µM FAM-MACTIDE and incubated at 37 °C. Forty µl aliquots were taken at the moment of mixing, 10, 30, 60, 180 and 1440 minutes. Eighty µl methanol was added to each aliquot and it was immediately stored in -80 °C until analysis later on the same or following day. For analysis the samples were centrifuged at 21 000 × g for 10 min at +4 °C. The supernatant was transferred into liquid chromatography (Agilent 1200 series) autosampler and maintained at +4 °C. Ten µl was injected and separation was achieved with a C18 column (Kinetex 2.6 µm EVO C18 100×4.6 mm, Phenomenex). The chromatography gradient started with 5 min 5 % acetonitrile in water, followed by linear increase to 100 % acetonitrile in 20 min and finally 20 min isocratic flow of 100 % acetonitrile. Both eluents contained 0.2 % formic acid. Enhanced resolution scan for peptides with 1, 2 or 3 charges was done. Additionally, all m/z values in 50-2000 range were scanned for degradation product search. Potential product signals were subjected to fragmentation analysis. Statistics was done in Graphpad Prism 5.0.

### Quartz crystal microbalance

The Quartz Crystal Microbalance (QCM) used was QCM200 system from Stanford Research Systems (Sunnyvale, CA, USA). The quartz crystals used have 5MHz resonant frequency and are deposited with a layer of Cr/Au (Cat # Q100RX1, pin 6-613, Stanford Research Systems). The crystal was mounted on the cell, washed with isopropanol, ethanol and mQ water. Then, it was incubated with 20mM Mercapto-propanesulfonate (MPS, Cat #251682, Sigma-Aldrich) in 10mM H₂SO₄ for 30 minutes, washed with mQ and then incubated with a 10µM (in monomer) of Poly (allylamine hydrochloride, Mw: 50000, PAH, Cat # 283223, Sigma-Aldrich) in mQ at pH 8.5 during 10 min and later washed with mQ. Then, a baseline with 500µL of mQ at pH 8 was recorded, the measurement was paused and then 5µL of a 1mg/mL solution in PBS were added (human recombinant CD206, Cat # 2534-MR-050/CF from R&D systems, reconstituted with 50µL of mQ); final concentration of CD206 in the cell: 0.01 mg/mL. Of note, the isoelectric point of CD206 is 6.3. For PAH-BSA multilayer, Bovine Serum Albumin (BSA, Cat # A7906, Sigma-Aldrich) was deposited, after measuring a baseline in mQ, at a concentration of 0.01mg/mL in mQ for 10 minutes, and then washed with mQ. For peptides, 500µL of PBS were placed in the cell and the baseline was recorded, the measurement was then paused and 50µL of a 100µM solution of peptides in PBS were gently deposited on the cell and the measurement was resumed. Then, the measurement was paused, the solution removed and replaced with 500µL of new PBS and the measurement resumed. The association and dissociation curves were fitted using TraceDrawer software (Ridgeview Instruments AB), to obtain the association constant ka, the dissociation constant kd and the affinity constant K_{D}.

## Claims

1. A peptide that comprises the amino acid sequence SEQ ID NO: 1: CTKSIPPICSPGAK.

2. The peptide according to claim 1, wherein the peptide is cyclic.

3. The peptide according to claim 1 or 2, wherein the cysteine at position 1 and the cysteine at position 9 are linked through a disulfide bond.

4. The peptide according to any one of claims 1 to 3, wherein the peptide has a length of no more than 50 amino acids.

5. The peptide according to any one of claim 1 to 4, wherein said peptide comprises the amino acid sequence SEQ ID NO: 2: GCTKSIPPICSPGAK.

6. A conjugate that comprises the peptide according to any one of claims 1 to 5 linked to a detectable agent or a therapeutic agent.

7. The conjugate according to claim 6, wherein the detectable agent is selected from the list consisting of a fluorophore, a radionuclide, an enzyme, a metal, barium sulphate, a ferromagnetic compound, a paramagnetic compound, a diamagnetic compound and any combination thereof.

8. The conjugate according to claim 6, wherein the therapeutic agent is selected from the list consisting of a photosensitizer, a small molecule, a DNA, a RNA, a peptide, a protein, a chemotherapeutic agent, a radiopharmaceutical compound, an antibody or any combination thereof.

9. The conjugate according to claim 6 or 8, wherein the therapeutic agent is a photosensitizer selected from the list consisting of verteporfin, 5-aminolevulinic acid (5-ALA), chlorin e6, IRDye700DX and Porfimer sodium.

10. The conjugate according to any one of claims 6, 8 or 9, wherein the therapeutic agent is verteporfin.

11. A pharmaceutical composition that comprises:
- the peptide according to any one of claims 1 to 5, or
- the conjugate according to any one of claims 6 to 10.

12. The peptide according to any one of claims 1 to 5, the conjugate according to any one of claims 6 to 10, or the pharmaceutical composition according to claim 11, for use as a medicament.

13. The peptide according to any one of claims 1 to 5, the conjugate according to any one of claims 6 to 10 or the pharmaceutical composition according to claim 11 for use in a method for the treatment and/or prevention of cancer.

14. The peptide according to any one of claims 1 to 5, the conjugate according to any one of claims 6 to 10 or the pharmaceutical composition according to claim 11 for use in a method for the treatment and/or prevention of an infection caused by a protozoon belonging to the genus *Leishmania.*

15. The peptide according to any one of claims 1 to 5, the conjugate according to any one of claims 6 to 10 or the pharmaceutical composition according to claim 11 for use in the detection, diagnosis and/or monitoring of cancer in a subject.

16. Use of the peptide according to any one of claims 1 to 5 or the conjugate according to any one of claims 6 to 10 for the *in vitro* detection of CD206.

17. An *in vitro* method for detecting CD206 in a biological sample that comprises:
a) contacting the biological sample with the peptide according to any one of claims 1 to 5 or the conjugate according to any one of claims 6 to 10, and
b) detecting the presence of the claims 1 to 5 or the conjugate according to any one of claims 6 to 10 bound to the biological sample.

18. Use *in vitro* of the peptide according to any one of claims 1 to 5, or the conjugate according to any one of claims 6 to 10 for promoting the conversion of a M2-macrophage to M1-macrophage.

19. An *in vitro* method for promoting the conversion of a M2-macrophage into M1-macrophage that comprises:
a) contacting the peptide according to any one of claims 1 to 5 or the conjugate according to any one of claims 6 to 10 with the M2-macrophage or a biological sample comprising the M2-macrophage.
